# EUROPÄISCHE PATENTSCHRIFT

(11) **EP 0 432 260 B1**
(45) Veröffentlichungstag und Bekanntmachung des Hinweises auf die Patenterteilung: **12.07.1995**
(21) Anmeldenummer: 90911602.2
(22) Anmeldetag: 04.07.1990
(51) Int. Cl.: A61B 17/14

(54) **VORRICHTUNG ZUM FÜHREN EINER INNENSÄGE ZUR OSTEOTOMIE LANGER RÖHRENKNOCHEN**
DEVICE FOR GUIDING AN OSTEOTOME DURING OSTEOTOMY OF LONG BONES
DISPOSITIF DE GUIDAGE D'UN OSTEOTOME POUR OSTEOTOMIE D'OS LONGS

(30) Priorität: 04.07.1989 DE 3921973
(43) Veröffentlichungstag der Anmeldung: 19.06.1991
(73) Patentinhaber: Baumgart, Rainer, Dipl.-Ing. Dr. med., D-81479 München (DE); BETZ, Augustin, D-82319 Starnberg (DE)
(72) Erfinder: Baumgart, Rainer, Dipl.-Ing. Dr. med., D-81479 München (DE); BETZ, Augustin, D-82319 Starnberg (DE)
(74) Vertreter: Finck, Dieter, Dr.Ing.
(86) Internationale Anmeldenummer: EP9001079
(87) Internationale Veröffentlichungsnummer: WO9100061

(56) Entgegenhaltungen:
- DE-B- 1 288 241
- US-A- 3 214 869
- US-A- 3 472 229
- MEYERS ENZYKLOPÄDISCHES LEXIKON Bd.7, Bibliographisches Institut A.G. Mannheim, 1973
- LUEGER, Lexikon der Technik Bd.15 1971 Deutsche Verlagsanstalt GmbH, Stuttgart

## Beschreibung

Die Erfindung betrifft eine Vorrichtung zum Führen einer Innensäge zur Osteotomie langer Röhrenknochen mit einem langgestreckten Schaft, in dem eine zu seiner Längsachse parallele exzentrische Bohrung für die Aufnahme einer die Innensäge tragenden Welle ausgebildet ist.

Es ist bereits bekannt, Röhrenknochen von außen durch die Knochenhaut hindurch oder von innen vom Markraum aus zu durchtrennen, wenn die Röhrenknochen beispielsweise verlängert oder verkürzt werden sollen, wenn Knochensegmentverschiebungen erforderlich sind oder wenn Achsenkorrekturen durchgeführt werden sollen.

Die Osteotomie von außen wird in der Regel mit einer oszillierenden Säge durchgeführt (DE-A- 1491219). Nach Freilegung des Knochens wirdzunächst das Periost und anschließend die Kortikalis durchtrennt. Durch die Durchtrennung des gefäßreichen, den Knochen vital versorgenden Periosts, können im Heilprozeß Vitalitätsstörungen auftreten.

Bei der Osteotomie vom aufgebohrten Markraum aus, also von innen, bleibt der den Röhrenknochen umgebende Periostschlauch weitgehend unversehrt, so daß Vitalitätsstörungen im Bereich der Knochentrennfuge nicht zu erwarten sind. Die Osteotomie vom Markraum aus ist vor allem dann zweckmäßig, wenn eine Marknagelung vorgenommen werden soll. Der Zugangsweg zur Einführung des Marknagels ist hierbei der gleiche wie er zur Einführung der Innensäge verwendet wird, so daß im Bereich der Osteotomie nicht nur der Periostschlauch, sondern auch die Weichteile intakt sind.

Bei der Osteotomie von innen wird gemäß der gattungsgemäßen Vorrichtung nach der US-A-3 472 229 ein kreisförmiges Sägeblatt verwendet, das auf einer zentralen Welle sitzt, die sich durch die exzentrische Bohrung im Schaft erstreckt, der zur Führung der Welle einen eine dünne gebogene Brücke bildenden, von seinem Umfangsabschnitt abstehenden Fortsatz hat, an den sich ein kurzer Endabschnitt mit exzentrischer Lagerbohrung anschließt. Das Sägeblatt wird bei dieser bekannten Vorrichtung gemeinsam mit der Führung in den Markraum eingebracht. Erst dann beginnt der Sägevorgang. Der Anpreßwinkel des Sägeblatts in die Kortikalis vom Lumen des Röhrenknochens aus ist kleiner als 90°, wodurch das Sägeblatt beim Sägen axial stark belastet wird, so daß es sich schirmförmig verformt. Dadurch ist die Ausführung eines sauberen Sägeschnitts nicht möglich. Es hat sich gezeigt, daß die mit den bekannten Führungssystemen erzielten Einschnitte nicht geschlossen sind, sondern schraubenförmig und verschliffen verlaufen.

Aus der DE-12 88 241 B ist eine Vorrichtung zum Hinterschneiden von Knochen mit einem durch eine Bohrung einführbaren, am Ende einer langen, vorzugsweise biegsamen, Antriebswelle befestigten, von der Bedienungsseite her spreizbaren Schneidwerkzeug bekannt, bei welcher zum radialen Durchtrennen von Röhrenknochen eine gesonderte, am Ort des Schneidwerkzeugs Andruckkraft entfaltende Einrichtung zur Verlagerung des Schneidwerkzeugs gegen die Knochenwand vorgesehen ist.

Die der Erfindung zugrundeliegende Aufgabe besteht nun darin, die Vorrichtung zum Führen einer Innensäge zur Osteotomie langer Röhrenknochen so auszubilden, daß sich eine exakte kreisringförmige Kortikalisdurchtrennung vom Markraum aus an jeder beliebigen Stelle eines Röhrenknochens ohne wesentliche Beschädigung der Knochenhaut erreichen läßt.

Diese Aufgabe wird ausgehend von der Vorrichtung der gattungsgemäßen Art durch einen Dorn, der von einem Ende des Schaftes zentrisch absteht und in dessen Umfangsfläche eine zu seiner Achse parallele Längsnut ausgespart ist, die sich in der fluchtend zu ihr ausgerichteten exzentrischen Bohrung in dem Schaft fortsetzt, und durch eine auf dem Dorn längsverschiebliche und fixierbare Distanzhülse gelöst.

Dabei hat die Längsnut zweckmäßigerweise wenigstens bodenseitig einen kreisförmigen Querschnitt, dessen Radius dem der exzentrischen Bohrung im Schaft entspricht. Damit das Sägeblatt bis zur maximalen Tiefe in die Kortikalis einsägen kann, entspricht dabei die Tiefe der Längsnut dem Durchmesser der exzentrischen Bohrung bzw. der in ihr geführten Welle.

Der Schaft kann dabei zylindrisch ausgebildet sein und erstreckt sich koaxial zum Dorn.

In den aufgebohrten Markraum wird das Sägeblatt mit seiner zentralen Welle bis zur geplanten Osteotomiestelle eingeführt. Anschließend wird die Welle in die Längsnut im Dorn und durch die Bohrung im Schaft hindurchgeführt. Unter Rotation der Säge wird nun die Vorrichtung in den Markraum geschoben, wodurch sich das Sägeblatt in die Kortikalis einsägt. Die Endstellung der Vorrichtung ist durch die vorher entsprechend verschobene und fixierte Distanzhülse vorgegeben. Anschließend wird die Vorrichtung gedreht, wodurch eine einwandfreie, radiale Führung des Sägeblatts gewährleistet ist. Mit Hilfe der erfindungsgemäßen Vorrichtung wird somit die zunächst zentral bezüglich des Markraums angeordnete Welle der Innensäge im Markraum radial nach außen in die Längsaussparung im Dorn und die Bohrung im Schaft verschoben und dann zusammen mit dem Schaft und dem Dorn gedreht, deren Achse sich nun zentral durch den Markraum erstreckt. Da die Antriebswelle vollständig in der Längsnut im Dorn aufgenommen ist, kann das Sägeblatt nahezu über seine radiale Erstreckung zwischen Umfang und seiner Welle in die Kortikalis eindringen.

Wenn die Vorrichtung so ausgestaltet ist, daß der Dorn auf seiner Längserstreckung in einem Zwischenbereich einen über den Boden seiner Längsnut hinaus reduzierten Querschnitt hat, fehlt in diesem Zwischenbereich die Führung der Welle, wodurch sich die Vorrichtung auch in einem gekrümmten Markraum einsetzen läßt.

Hierbei läßt sich eine stabile Wellenführung dann verwirklichen, wenn die Längsnut im verjüngungsseitigen Abschnitt des Dorns als eine seine Umfangsfläche tangierende Bohrung ausgebildet ist.

Die Vorrichtung läßt sich dann besonders einfach in den Markkanal eintreiben, wenn sich das dem Schaft gegenüberliegende Ende des Dorns verjüngt.

Zweckmäßigerweise ist an dem dem Dorn gegenüberliegenden Ende des Schaftes eine Einrichtung für den Eingriff mit einem den Schaft drehenden Werkzeug vorgesehen, beispielsweise in Form eines an der Umfangsfläche des Schaftes ausgebildeten Sechskants für das Angreifen eines entsprechenden Schlüssels, oder in Form einer Bohrung zum Einführen eines radialen Hebels.

Ferner können sich durch den Schaft und den Dorn erstreckende Känale für die Zuführung eines Kühl- und/oder Spülfluids und für dessen Abführung zusammen mit dem Sägeabraum vorgesehen werden. Dabei wird das Kühlfluid beim Auftreffen auf das Blatt der Innensäge durch die Zentrifugalkraft in den Sägespalt geschleudert und spült dadurch die ausgesägten Späne zum Abführkanal.

Anhand von Zeichnungen werden Ausführungsbeispiele der Erfindung näher erläutert. Es zeigt:
Fig. 1 perspektivisch eine erste Ausführungsform der Vorrichtung,
Fig. 2 die Vorrichtung von Fig. 1 mit Innensäge eingeführt in einen Röhrenknochen und
Fig. 3 perspektivisch eine zweite Ausführungsform der Vorrichtung.

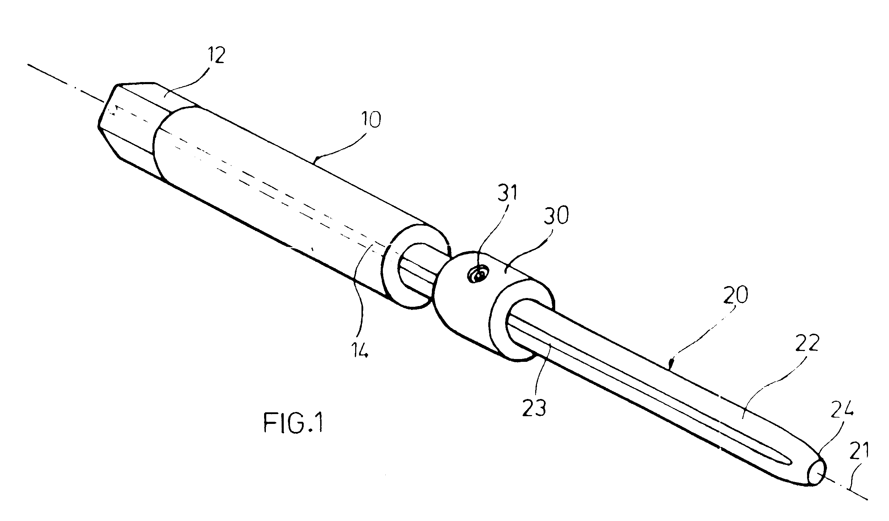

Die in den Figuren 1 bis 3 gezeigte Vorrichtung besteht aus einem zylindrischen Schaft 10, an dessen einem Ende ein Sechskant 12 ausgebildet ist und an dessen anderem Ende koaxial ein zylindrischer Dorn 20 absteht, dessen Durchmesser geringer ist als der des Schaftes 10. Auf dem Dorn 20 ist eine Distanzhülse 30 längsverschiebbar angeordnet. Die Distanzhülse 30 ist mit Hilfe einer Madenimbusschraube 31 in einer eingestellten axialen Position fixierbar. Der Schaft 10, der Dorn 20 und die Distanzhüle 30 haben eine gemeinsame Achse 21. Der Dorn 20 hat an seinem freien Ende eine Verjüngung 24.

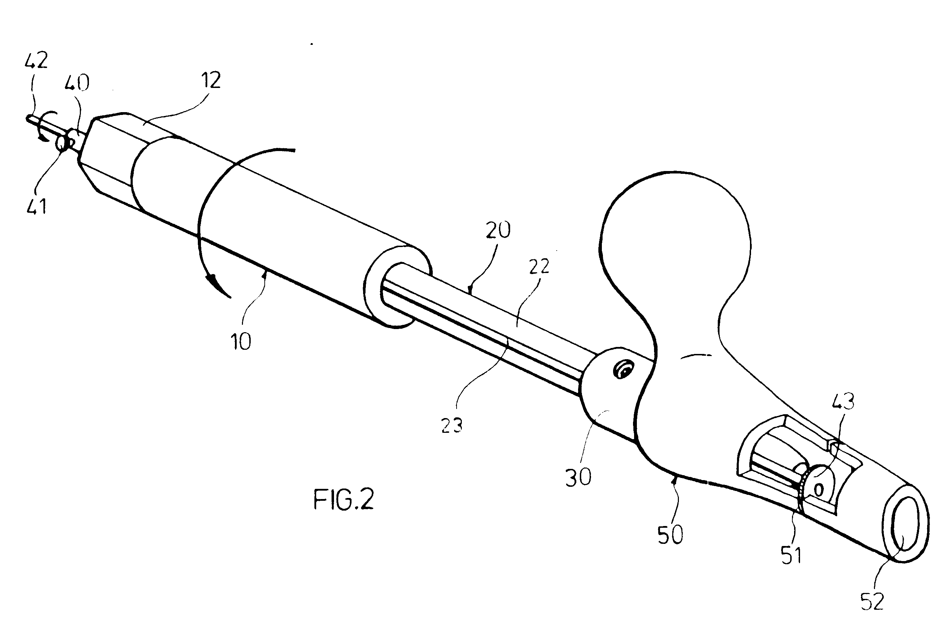

Der Dorn 20 hat eine Umfangsfläche 22, in der eine zur Achse 21 parallele Längsnut 23 ausgespart ist. Fluchtend zu der Längsnut 23 erstreckt sich durch den Schaft 10 eine zur Achse 21 parallele Längsaussparung in Form einer Bohrung 14. Die Bohrung 14 und die Längsnut 23 sind so bemessen, daß in ihnen eine in Figur 2 gezeigte Welle 42 einer Innensäge 43 aufgenommen werden kann. Für diesen Zweck hat die Längsnut 23 einen halbkreisförmigen Boden und sich zu der Umfangsfläche 22 hin erstreckende Seitenwände.

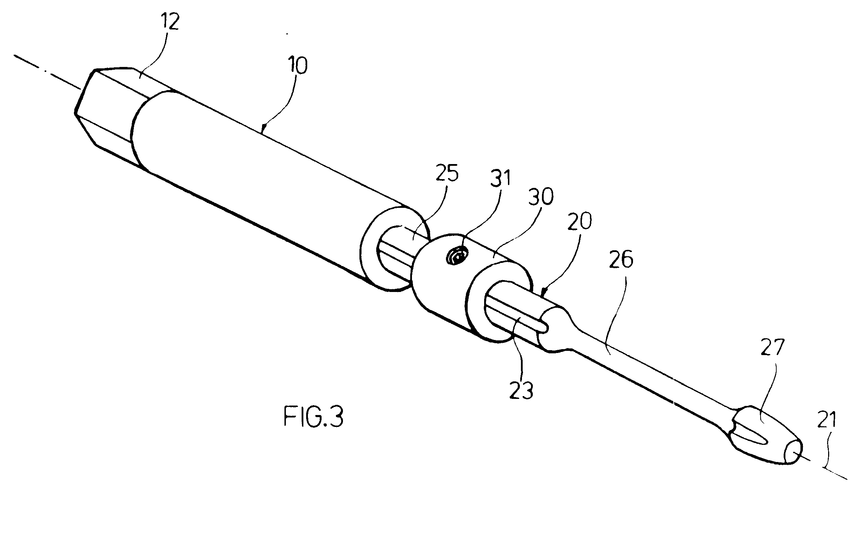

Bei der in Figur 3 gezeigten Ausführungsform hat der Dorn 20 einen schaftseitigen Abschnitt 25 und einen verjüngungsseitigen Abschnitt 27, längs derer sich die Längsnut 23 erstreckt. Zwischen dem schaftseitigen Abschnitt 25 und dem verjüngungsseitigen Abschnitt 27 befindet sich ein Zwischenabschnitt 26 mit einem derart reduzierten Querschnitt, daß dort die Längsnut 23 nicht mehr vorhanden ist.

Wenn bei dem in Fig. 2 gezeigten Röhrenknochen 50 der Markraum 52 in bekannter Weise aufgebohrt ist, wird das an der Welle 42 sitzende Blatt der Innensäge 43 bis zu der Stelle eingeführt, wo die Osteotomie ausgeführt werden soll. Dann wird die Welle 42 in die Längsnut 23 des Dorns 20 und durch die Bohrung 14 im Schaft 10 und aus diesem heraus geführt. Nach dem Verbinden mit einem nicht gezeigten Antriebsmotor wird die Vorrichtung bei drehender Welle 42 bei unveränderter axialer Position des Blatts der Innensäge 43 so weit in den Röhrenknochen 50 vorgeschoben, bis die Distanzhülse 30 am Röhrenknochen anliegt, wobei diese Anschlagstellung vorher eingestellt worden ist. Während des Einschiebens der Vorrichtung in den Röhrenknochen 50 wird die Welle 42 bereits deshalb angetrieben, damit sich das Blatt der Innensäge 43 an einer Stelle in die Kortikalis unter Bildung eines Einschnitts 51 einsägt, dessen Schnittiefe aufgrund der konturnahen Führung der Welle 42 der maximal möglichen Schnittiefe des Blatts der Innensäge 43 entspricht. Wenn die Distanzhülse 30 an der Eintrittstelle in den Röhrenknochen anliegt, wird auf der Welle 42 eine Klemmhülse 40 bis an den Sechskant 12 des Schaftes 10 vorgeschoben und mit Hilfe einer Klemmschraube 41 fixiert. Mit Hilfe eines am Sechskant 12 des Schaftes 10 angreifenden Gabelschlüssels kann nun bei angetriebener Innensäge 43 die Vorrichtung um 360° langsam gedreht werden, wobei das Blatt der Innensäge 43 einen geschlossenen ringförmigen Einschnitt 51 in die Kortikalis des Röhrenknochens 50 von seinem Markraum aus einsägt, ohne dabei die umgebende Knochenhaut wesentlich zu verletzen.

## Patentansprüche

1. Vorrichtung zum Führen einer Innensäge (43) zur Osteotomie langer Röhrenknochen (50) mit einem langgestreckten Schaft (10), in dem eine zu seiner Längsachse parallele exzentrische Bohrung (14) für die Aufnahme einer die Innensäge (43) tragenden Welle (42) ausgebildet ist, **gekennzeichnet** durch einen Dorn (20), der von einem Ende des Schafts (10) zentrisch absteht und in dessen Umfangsfläche eine zu seiner Achse (21) parallele Längsnut (23) ausgespart ist, die sich in der fluchtend zu ihr ausgerichteten exzentrischen Bohrung (14) im Schaft (10) fortsetzt, und durch eine auf dem Dorn (20) längsverschiebliche und fixierbare Distanzhülse (30).

2. Vorrichtung nach Anspruch 1, dadurch **gekennzeichnet,** daß die Längsnut (23) wenigstens bodenseitig einen kreisförmigen Querschnitt hat, dessen Radius dem der exzentrischen Bohrung (14) im Schaft (10) entspricht, und daß die Tiefe der Längsnut (23) dem Durchmesser der exzentrischen Bohrung (14) entspricht.

3. Vorrichtung nach Anspruch 1 oder 2, **dadurch gekennzeichnet,** daß der Schaft (10) zylindrisch und koaxial zum Dorn (20) ausgebildet ist.

4. Vorrichtung nach einem der vorhergehenden Ansprüche, **dadurch gekennzeichnet,** daß der Dorn (20) auf seiner Längserstreckung in einem Zwischenbereich (26) einen über den Boden seiner Längsnut (23) hinaus reduzierten Querschnitt hat.

5. Vorrichtung nach Anspruch 4, **dadurch gekennzeichnet,** daß die Längsnut (23) im verjüngungsseitigen Abschnitt (27) des Dorns (20) eine seine Umfangsfläche (22) tangierende Bohrung ist.

6. Vorrichtung nach einem der vorhergehenden Ansprüche, **dadurch gekennzeichnet,** daß sich das dem Schaft (10) gegenüberliegende Ende des Dorns (20) verjüngt.

7. Vorrichtung nach einem der vorhergehenden Ansprüche, **dadurch gekennzeichnet,** daß sich an dem dem Dorn (20) gegenüberliegenden Ende des Schaftes (10) eine Einrichtung (12) für den Eingriff mit einem den Schaft (10) drehenden Werkzeug vorgesehen ist.

8. Vorrichtung nach Anspruch 7, **dadurch gekennzeichnet,** daß die Einrichtung (12) ein an der Umfangsfläche des Schaftes (10) ausgebildeter Sechskant ist.

9. Vorrichtung nach einem der vorhergehenden Ansprüche, **gekennzeichnet** durch sich durch den Schaft (10) und den Dorn (20) erstreckende Kanäle für die Zuführung eines Kühl- und/oder Spülfluids und für dessen Abführung zusammen mit dem Sägeabraum.

## Claims

1. Device for guiding an internal saw (43) for the osteotomy of long tubular bones (50), having an elongated shank (10) in which is formed, parallel to its longitudinal axis, an eccentric bore (14) for accommodating a shaft (42) carrying the internal saw (43), characterised by a spindle (20) which projects centrally from one end of the shank (10) and in the circumferential face of which is recessed, parallel to its axis (21), a longitudinal slot (23) which extends in the eccentric bore (14) which is provided in the shank (10) and is aligned with the longitudinal slot (23), and by a distance sleeve (30) which can be displaced longitudinally on and fixed in position on the spindle (20).

2. Device according to Claim 1, characterised in that the longitudinal slot (23) has, at least on the base side, a circular cross-section of which the radius corresponds to that of the eccentric bore (14) in the shank (10), and in that the depth of the longitudinal slot (23) corresponds to the diameter of the eccentric bore (14).

3. Device according to Claim 1 or 2, characterised in that the shank (10) is cylindrical and coaxial with the spindle (20).

4. Device according to any one of the preceding claims, characterised in that the spindle (20) has, on its longitudinal extent, in an intermediate area (26), a cross-section which is reduced beyond the base of its longitudinal slot (23).

5. Device according to Claim 4, characterised in that the longitudinal slot (23) is, in the portion (27) of the spindle (20) next to the tapered portion, a bore adjacent to the circumferential face (22) of the spindle (20).

6. Device according to any one of the preceding claims, characterised in that the end of the spindle (20) lying opposite the shank (10) tapers.

7. Device according to any one of the preceding claims, characterised in that a device (12) for engagement with a tool turning the shank (10) is provided on the end of the shank (10) lying opposite the spindle (20).

8. Device according to Claim 7, characterised in that the device (12) is a hexagonal member formed on the circumferential face of the shank (10).

9. Device according to any one of the preceding claims, characterised by channels extending through the shank (10) and the spindle (20) for the supply of a cooling and/or rinsing fluid and for the removal thereof together with the sawing waste.

## Revendications

1. Dispositif de guidage d'un ostéotome (43) pour l'ostéotomie d'os longs (50), comprenant une tige allongée (10), dans laquelle est formé un perçage excentrique (14) parallèle à son axe longitudinal pour le logement d'un arbre (42) portant l'ostéotome (43), caractérisé par un mandrin (20) faisant saillie au centre d'une extrémité de la tige (10) et dans la périphérie duquel est ménagée une rainure longitudinale (23) parallèle à son axe (21), laquelle rainure se prolonge dans le perçage excentrique (14) de la tige (10), aligné sur ladite rainure, et par une douille d'écartement (30) susceptible d'être déplacée longitudinalement et bloquée sur le mandrin (20).

2. Dispositif selon la revendication 1, caractérisé en ce que la rainure longitudinale (23) présente au moins du côté du fond une section circulaire dont le rayon correspond à celui du perçage excentrique (14) de la tige (10), et en ce que la profondeur de la rainure longitudinale (23) correspond au diamètre du perçage excentrique (14).

3. Dispositif selon la revendication 1 ou 2, caractérisé en ce que la tige (10) est cylindrique et coaxiale par rapport au mandrin (20).

4. Dispositif selon l'une quelconque des revendications précédentes, caractérisé en ce que le mandrin (20) présente sur son allongement longitudinal dans une zone intermédiaire (26) une section réduite au-delà du fond de sa rainure longitudinale (23).

5. Dispositif selon la revendication 4, caractérisé en ce que la rainure longitudinale (23) constitue dans la partie (27) du mandrin (20) du côté de la partie réduite un perçage qui est en contact avec la périphérie (22) du mandrin.

6. Dispositif selon l'une quelconque des revendications précédentes, caractérisé en ce que l'extrémité du mandrin (20) placée à l'opposé de la tige (10) s'amincit.

7. Dispositif selon l'une quelconque des revendications précédentes, caractérisé en ce qu'un dispositif (12) pour l'engrènement avec un outil faisant tourner la tige (10) est prévu sur l'extrémité de la tige (10) placée à l'opposé du mandrin (20).

8. Dispositif selon la revendication 7, caractérisé en ce que le dispositif (12) est un hexagone formé à la périphérie de la tige (10).

9. Dispositif selon l'une quelconque des revendications précédentes, caractérisé par des canaux qui traversent la tige (10) et le mandrin (20) et qui sont destinés à amener un liquide de refroidissement et/ou de rinçage et à l'évacuer avec la sciure.
